(19) Europäisches Patentamt
European Patent Office
Office européen des brevets

(11) Veröffentlichungsnummer: **0 120 239**
**A2**

(12) # EUROPÄISCHE PATENTANMELDUNG

(21) Anmeldenummer: **84101209.9**

(22) Anmeldetag: **07.02.84**

(51) Int. Cl.[3]: **C 07 D 233/60**
A 61 K 31/415
//C07D233/56, C07D303/22,
C07C149/00

(30) Priorität: **25.02.83 DE 3306646**

(43) Veröffentlichungstag der Anmeldung:
**03.10.84 Patentblatt 84/40**

(84) Benannte Vertragsstaaten:
**AT BE CH DE FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Gante, Joachim, Dr.**
**Stormstrasse 4,**
**D-6100 Darmstadt-Arheilgen(DE)**

(72) Erfinder: **Prücher, Helmut**
**Königsbergerstrasse 9**
**D-6148 Heppenheim(DE)**

(72) Erfinder: **Wahlig, Helmut, Dr.**
**Roemheldweg 16**
**D-6100 Darmstadt(DE)**

(72) Erfinder: **Rudolph, Volkmar, Dr.**
**Im Neutscher Grund 19**
**D-6104 Seeheim 3(DE)**

(54) **Schwefelhaltige Imidazolderivate.**

(57) Neue schwefelhaltige Imidazolderivate der allgemeinen Formel I

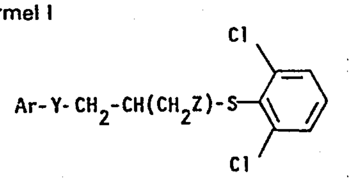

I

worin

AR einen unsubstituierten oder einen durch ein oder zwei Halogenatome substituierten Phenylrest,

Y O oder S und

Z einen I-Imidazolyl- oder einen 2-Methyl-1- imidazolylrest

bedeuten,

sowie ihre physiologisch unbedenklichen Säureadditionssalze wirken antimykotisch und antibakteriell.

EP 0 120 239 A2

# Schwefelhaltige Imidazolderivate

Die Erfindung betrifft neue schwefelhaltige Imidazolderivate der allgemeinen Formel I

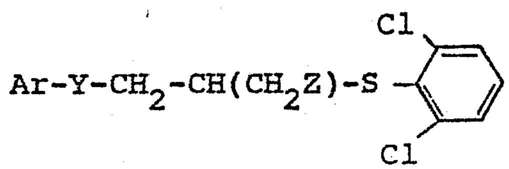

I

worin

Ar einen unsubstituierten oder einen durch ein oder zwei Halogenatome substituierten Phenylrest,

Y O oder S und

Z einen 1-Imidazolyl- oder einen 2-Methyl-1-imidazolylrest

bedeuten,

sowie ihre Säureadditionssalze.

Ähnliche Verbindungen sind aus der US-PS 4 036 970 bekannt. In dieser Patentschrift ist eine allgemeine Formel

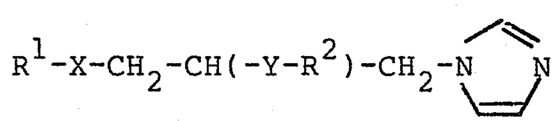

angegeben, worin $R^1$ auch Phenyl oder durch ein oder zwei Halogenatome substituiertes Phenyl, X auch O oder S, Y auch S und $R^2$ auch eine durch eine oder zwei Halogenatome substituierte Phenylgruppe bedeuten können. Obwohl dort jedoch Beispiele mit $R^2$ = 3,4-Dichlorphenyl und $R^2$ = 2,4-Dichlorphenyl angegeben sind, fehlt jeder Hinweis darauf, daß $R^2$ auch eine 2,6-Dichlorphenylgruppe und insbesondere $-Y-R^2$ auch

eine 2,6-Dichlorphenylthiogruppe bedeuten kann. Aus dieser Patentschrift kann der Fachmann daher keinen Hinweis auf die Verbindungen der vorliegenden Formel I entnehmen; diese Verbindungen werden durch die genannte US-PS nicht nahegelegt.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I bei guter Verträglichkeit wertvolle pharmakologische Eigenschaften besitzen. Insbesondere treten antimykotische und antibakterielle Wirkungen (in vitro und in vivo) auf, beispielsweise gegen Fadenpilze wie Microsporum audouini, Microsporum gypseum, Epidermophyton floccosum, Trichophyton rubrum, Trichophyton tonsurans, Trichophyton mentagrophytes, Blastomyces brasiliensis und Histoplasma capsulatum; Schimmelpilze wie Aspergillus fumigatus, Aspergillus niger und Scopulariopsis fusca; Hefen wie Candida albicans, Candida parapsilosis und Cryptococcus neoformans; Actinomyceten wie Nocardia minutissima und Nocardia asteroides; gramnegative und grampositive Bakterien wie Staphylococcus aureus, Streptococcus pyogenes, Streptococcus faecalis, Corynebacterium acnes, Erysipelothrix insidiosa, Proteus vulgaris, Proteus mirabilis, Salmonella choleraesuis, Pasteurella multocida, Pseudomonas aeruginosa, Mycobacterium ranae und Escherichia coli. Die Verbindungen wirken auch gegen systemische Pilzinfektionen; ferner treten Wirkungen gegen Protozoen, insbesondere gegen Trichomonaden auf, z.B. gegen Trichomonas vaginalis.

Diese Wirksamkeiten können z.B. nach der üblichen Agarverdünnungsmethodik in vitro ermittelt werden, aber auch in vivo, beispielsweise an Mäusen, Ratten oder Kaninchen.

Weiterhin zeigen sich antiphlogistische Wirkungen, die sich z.B. im Adjuvans-Arthritis-Test nach der Methode von Newbould (Brit. J. Pharmacol. 21. (1963) Seiten 127 - 136) an Ratten nachweisen lassen. Ferner zeigen sich antiarteriosklerotische, cholesterinspiegelsenkende (nachweisbar im Serum von Ratten nach der Methode von Levine et al., Automation in Analytical Chemistry, Technicon Symposium 1967, Mediad, New York, Seiten 25 - 28) und triglyceridspiegelsenkende Wirkungen (nachweisbar nach der Methode von Noble und Campbell, Clin. Chem. 16 (1970) Seiten 166 - 170). Weiterhin können analgetische, antipyretische, enzyminduzierende, fibrinolytische und thrombozyten-aggregationshemmende Wirkungen nach hierfür geläufigen Methoden beobachtet werden.

Die Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind Imidazolderivate der Formel I sowie ihre Salze.

In den oben angegebenen Resten bedeutet Ar vorzugsweise p-Chlorphenyl, ferner Phenyl, o-Chlorphenyl, 2,4- oder 2,6-Dichlorphenyl, ferner auch m-Chlorphenyl, 2,3-,

2,5-, 3,4- oder 3,5-Dichlorphenyl, o-, m- oder p-Fluorphenyl, 2,3-, 2,4-, 2,5-, 2,6-, 3,4- oder 3,5-Difluorphenyl, o-, m- oder p-Bromphenyl, 2,3-, 2,4-, 2,5-, 2,6-, 3,4- oder 3,5-Dibromphenyl, o-, m- oder p-Jodphenyl, 2,4-, 2,5-, 2,6- oder 3,5-Dijodphenyl, 2-Fluor-4-chlorphenyl, 2-Chlor-4-fluorphenyl, 2-Chlor-6-fluorphenyl, 2-Chlor-4-bromphenyl. Der Rest Y ist vorzugsweise O. Der Rest Z steht bevorzugt für 1-Imidazolyl.

Dementsprechend sind Gegenstand der Erfindung insbesondere diejenigen Verbindungen der Formel I, in denen mindestens einer der Reste Ar, Y und/oder Z eine der vorstehend angegebenen bevorzugten Bedeutungen hat.

Gegenstand der Erfindung ist ferner ein Verfahren zur Herstellung der Verbindungen der Formel I sowie ihrer Säureadditionssalze, dadurch gekennzeichnet, daß man eine Verbindung der allgemeinen Formel II

$$Ar-Y-CH_2-CH(CH_2Z)-X \qquad II$$

worin

X Cl, Br, J, OH oder funktionell abgewandeltes OH bedeutet und

Ar, Y und Z die angegebenen Bedeutungen haben

mit 2,6-Dichlorthiophenol oder einem seiner Salze umsetzt,

oder daß man eine Verbindung der allgemeinen Formel III

$$Ar-Y-CH_2-CH(CH_2X^1)-X^2 \qquad III$$

worin
der eine der Reste $X^1$ und $X^2$ X,
der andere dieser Reste - S 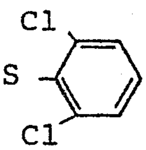 bedeutet und
Ar, X und Y die angegebenen Bedeutungen haben,
mit einem Imidazol der allgemeinen Formel H-Z (IV) oder einem seiner Metallderivate umsetzt,
oder daß man eine Verbindung der allgemeinen Formel V

$X-CH_2-CH(CH_2Z)-S$ 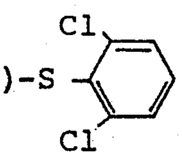 V

worin
X und Z die angegebenen Bedeutungen haben,
mit einer Verbindung der allgemeinen Formel VI

Ar-Y-H VI

worin
Ar und Y die angegebenen Bedeutungen haben
oder einem ihrer Salze umsetzt
und/oder daß man, falls erwünscht, eine Base der allgemeinen Formel I durch Behandeln mit einer Säure in eines ihrer Säureadditionssalze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der organischen Chemie, Georg-Thieme-Verlag, Stuttgart) beschrieben sind, und zwar unter den für die genannten Umsetzungen bekannten und geeigneten Reaktionsbedingungen. Dabei kann man auch von an sich bekannten, hier nicht näher erwähnten Varianten Gebrauch machen.

In allen vor- und nachstehenden allgemeinen Formeln haben Ar, Y und Z die bei Formel I angegebene Bedeutung, falls nicht ausdrücklich etwas anderes angegeben ist.

Die Ausgangsstoffe zur Herstellung der Verbindungen der Formel I sind teilweise bekannt. Sie können nach an sich bekannten Verfahren hergestellt werden. Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I sind vorzugsweise erhältlich durch Umsetzung einer Verbindung der Formel II mit 2,6-Dichlor-thiophenol oder einem seiner Salze, z.B. dem Na- oder K-Salz, das auch in situ aus dem Thiophenol und einer Base, z.B. Natrium- oder Kaliumhydroxid, gebildet werden kann.

In den Verbindungen der Formel II bedeutet X vorzugsweise Cl, Br oder J. Falls X eine funktionell abgewandelte OH-Gruppe bedeutet, so steht es vorzugsweise für

eine Alkyl- oder Arylsulfonyloxygruppe mit insbesondere bis zu 10 C-Atomen, z.B. für Methan-, Benzol- oder p-Toluolsulfonyloxy.

Die Ausgangsstoffe der Formel II sind in der Regel neu. Sie können z.B. hergestellt werden durch Reaktion von Epichlorhydrin mit Phenolaten bzw. Thiophenolaten der Formel Ar-Y-Na zu 1,2-Epoxy-3-ArY-propanen, Reaktion mit Imidazolen der Formel H-Z oder ihren Metallderivaten zu Hydroxyverbindungen der Formel $Ar-Y-CH_2-CHOH-CH_2-Z$ und, falls erwünscht, Umwandlung in die 2-Chlor- oder 2-Bromverbindung (mit $SOCl_2$ oder $PBr_3$) oder in einen entsprechenden Sulfonsäureester (z.B. in das Methansulfonat mit Methansulfonylchlorid).

Die Imidazolderivate der Formel II werden mit 2,6-Dichlorthiophenol oder dessen Salzen zweckmäßig bei Temperaturen zwischen etwa 0 und 100° in Gegenwart eines inerten Lösungsmittels, z.B. eines Alkohols wie Methanol, Ethanol, Isopropanol oder n-Butanol, eines Ethers wie Diethylether, Diisopropylether, Tetrahydrofuran oder Dioxan, eines Kohlenwasserstoffs wie Benzol, Toluol oder Xylol, eines Amids wie Dimethylformamid, eines Sulfoxids wie Dimethylsulfoxid oder eines Ketons wie Aceton umgesetzt.

Die Imidazolderivate der Formel I sind ferner erhältlich durch Reaktion eines Thioethers der Formel III mit einem Imidazol der Formel H-Z (IV) oder einem seiner Metallderivate, z.B. dem Na- oder K-Derivat.

Die Ausgangsstoffe der Formel III können z.B. hergestellt werden durch Reaktion der oben angegebenen 1,2-Epoxy-3-ArY-propane mit 2,6-Dichlorthiophenol und, falls erwünscht, Umwandlung der OH-Gruppe in das entsprechende Derivat, z.B. das Chlorid, Bromid oder Methansulfonat.

Sie umfassen Thioether der Formeln IIIa und IIIb:

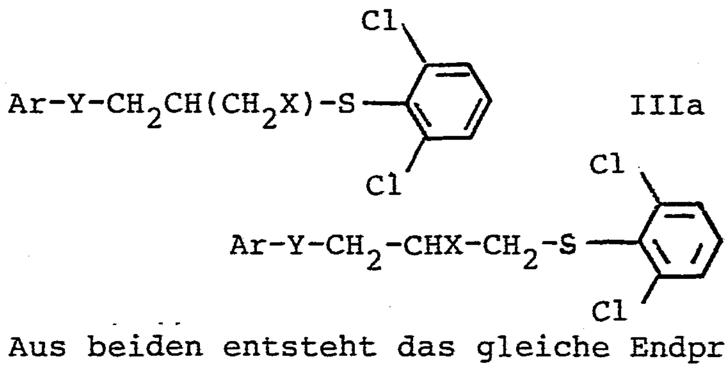

IIIa

IIIb

Aus beiden entsteht das gleiche Endprodukt I. Vermutlich verläuft die Reaktion über die Zwischenstufe des Sulfoniumsalzes VII

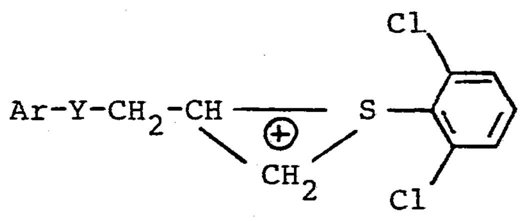 $X^{\ominus}$ VII

Die Ausgangsstoffe der Formel IV (Imidazol und 2-Methylimidazol) sind bekannt.

Die Umsetzung der Thioether der Formel III mit den Imidazolen der Formel IV erfolgt zweckmäßig bei Temperaturen zwischen etwa 0 und etwa 250°, vorzugsweise zwischen etwa 20 und 120°. Man kann ohne Lösungsmittel oder in Gegenwart eines der genannten inerten Lösungsmittel arbeiten. Falls erwünscht, kann ein Katalysator zugegen sein, z.B. Natriumamid, das auch aus Natrium und flüssigem Ammoniak in situ erzeugt werden kann, ferner Basen wie Natriumcarbonat, Kaliumcarbonat, Natriumbicarbonat oder Kaliumbicarbonat. Man verwendet zweckmäßig einen Überschuß der Verbindung der Formel IV; dieser Überschuß kann auch als Lösungsmittel dienen (in der Schmelze).

Die Verbindungen der Formel I können ferner durch Reaktion der Imidazolderivate der Formel V mit den Phenolen bzw. Thiophenolen der Formel VI erhalten werden.

Die Verbindungen der Formel V sind z.B. erhältlich durch Reaktion von 3-Brompropionsäure mit einem Imidazol der Formel H-Z (IV) zu einer 3-Z-propionsäure, Bromierung zu einer 2-Brom-3-Z-propionsäure, Reaktion mit Na-2,6-dichlorthiophenolat zu einer 2-(2,6-Dichlorphenylthio)-3-Z-propionsäure, Reduktion mit $LiAlH_4$ zu einem Alkohol der Formel V (X = OH) und, falls erwünscht, weitere Reaktion mit $SOCl_2$, $PBr_3$ oder einem Sulfonylchlorid. Die Verbindungen der Formel VI sind in der Regel bekannt.

Vorzugsweise wird das Phenol bzw. Thiophenol der Formel VI zunächst in ein Salz übergeführt, insbesondere ein Metallsalz, z.B. ein Alkalimetallsalz (Li-, Na- oder K-Salz). Man kann das Phenol bzw. Thiophenol mit einem metallsalz-bildenden Reagenz umsetzen, z.B. einem Alkalimetall (z.B. Na), einem Alkalimetallhydrid oder -amid (z.B. LiH oder NaH; $NaNH_2$ oder $KNH_2$), einem Alkalimetallalkoholat (worin der Alkohol-Teil vorzugsweise 1 - 4 C-Atome besitzt, z.B. Lithium-, Natrium- oder Kaliummethylat, -ethylat oder tert.-butylat), einer Organometallverbindung (z.B. Butyllithium, Phenyllithium oder Phenylnatrium), einem Metallhydroxid, -carbonat oder -bicarbonat (z.B. des Li, Na, K oder Ca). Die Herstellung des Phenolats bzw. Thiophenolats wird

vorteilhaft in Gegenwart eines der genannten Lösungsmittel oder eines Gemisches dieser Lösungsmittel vorgenommen.

Die Reaktion des Phenolats bzw. Thiophenolats mit der Verbindung V erfolgt vorzugsweise in Gegenwart eines Verdünnungsmittels, z.B. desjenigen Lösungsmittels, das für die Herstellung des Phenolats bzw. Thiophenolats verwendet worden ist, das jedoch durch ein anderes Lösungsmittel ersetzt oder mit einem solchen verdünnt sein kann. Die Umsetzung wird vorzugsweise bei Temperaturen zwischen 20 und 120° durchgeführt.

Das Phenolat bzw. Thiophenolat kann auch in situ gebildet werden. In diesem Falle läßt man das Phenol bzw. Thiophenol VI und die Verbindung V miteinander in Gegenwart einer Base reagieren. Eine besonders bevorzugte Methode besteht darin, daß man die Verbindungen V und VI zusammen mit einer alkoholischen Natriumhydroxidlösung etwa 5 bis 15 Stunden erhitzt.

Eine erhaltene Base der Formel I kann mit einer Säure in üblicher Weise in das zugehörige Säureadditionssalz übergeführt werden. Für diese Umsetzung eignen sich vorzugsweise starke Säure, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Malonsäure, Bernsteinsäure, Pimelinsäure, Fumarsäure, Maleinsäure, Milchsäure, Weinsäure, Apfelsäure, Salicylsäure, 2- oder 3-Phenylpropionsäure, Citronensäure, Gluconsäure, Ascorbinsäure, Methan- und Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit Säuren, die physiologisch nicht unbedenklich sind (z.B. die Pikrate), eignen sich zur Isolierung und Aufreinigung der Basen der Formel I.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I enthalten ein Asymmetriezentrum. Sie liegen gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie ß-Camphersulfonsäure, ferner Camphansäure.

Natürlich ist es auch möglich, optisch-aktive Verbindungen der Formel I nach den oben beschriebenen Methoden zu erhalten, indem man Ausgangsstoffe verwendet, die bereits optisch-aktiv sind.

Gegenstand der Erfindung ist ferner die Verwendung der neuen Verbindungen der Formel I und ihrer physiologisch unbedenklichen Säureadditionssalze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls zusammen mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Säureadditionssalze.

Diese Zubereitungen können als Arzneimittel in der Human- und Veterinärmedizin verwendet werden. Als Trägerstoffe kommen organische oder anorganische Substanzen in Frage, die sich insbesondere für die enterale (z.B. orale), parenterale oder topische Applikation eignen und mit den neuen Verbindungen nicht reagieren, beispielsweise Wasser, pflanzliche Öle, Kohlenwasserstoffe wie alkylierte Naphthaline, halogenierte Kohlenwasserstoffe wie $CF_2Cl_2$ (z.B. für Aerosole), Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur intravaginalen Ovula, zur parenteralen Anwendung Lösungen, vorzugsweise ölige oder wässerige Lösungen, ferner Suspensionen, Emulsionen oder Implantate, für die topikale

Anwendung Lösungen, Lotionen, Emulsionen, Sprays (Aerosole), Salben, Cremes, Pasten oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Antibiotika, Vitamine und/oder andere Antimykotika.

Die neuen Verbindungen werden in der Regel in Analogie zu bekannten, im Handel befindlichen Antimykotika (z.B. Clotrimazol oder Miconazol) verabreicht, vorzugsweise in Dosierungen zwischen etwa 2 und 600 mg, insbesondere zwischen 5 und 200 mg pro Dosierungseinheit. Die tägliche Dosierung liegt vorzugsweise zwischen etwa 0,1 und 20 mg/kg Körpergewicht. Dabei sind die niedrigen Dosierungen vorzugsweise für die parenterale, die höheren für die orale Applikation maßgeblich. Bevorzugt ist eine orale oder parenterale Applikation, insbesondere gegen systemische Candida-Infektionen, z.B. Infektionen mit Candida albicans, ferner gegen die südamerikansiche Blastomykose und sämtliche Organmykosen, vor allem verursacht durch Candida-Arten, Aktinomyzeten, Schimmelpilze, sonstige Pilze und Infektionen durch Bakterien, insbesondere grampositive Bakterien.
Bei der topischen Applikation in Kombination mit dafür geeigneten Trägerstoffen kann eine hohe Aktivität über einen weiten Verdünnungsbereich festgestellt werden. Beispielsweise zeigen sich Konzentrationen des Wirk-

stoffs zwischen etwa 0,1 und 10 Gewichtsprozent, bezogen auf das Gewicht des verwendeten Präparats, als wirksam für die Bekämpfung von Pilzen oder Bakterien. Bevorzugt sind Konzentrationen von etwa 1 bis 3 Gewichtsprozent.

Sofern die neuen Verbindungen als Antiphlogistika oder Lipidsenker verwendet werden, ist ihre orale Applikation bevorzugt. Sie werden dann in der Regel in Analogie zu bekannten Antiphlogistika (z.B. Indometacin) oder Lipidsenkern (z.B. Bezafibrat) verabreicht, vorzugsweise in Dosierungen zwischen etwa 5 und 500 mg, insbesondere zwischen 20 und 200 mg pro Dosierungseinheit.

Die tägliche Dosierung liegt vorzugsweise zwischen etwa 0,2 und 20 mg/kg Körpergewicht. Die spezielle Dosis für jeden Patienten hängt jedoch von den verschiedensten Faktoren ab, z.B. von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung. So können in Einzelfällen auch höhere oder niedrigere Konzentrationen bzw. Dosierungen als die angegebenen angewendet werden.

In den nachfolgenden Beispielen bedeutet "übliche Aufarbeitung": man gibt, falls erforderlich, Wasser oder verdünnte Natronlauge hinzu, extrahiert mit einem organischen Lösungsmittel, das mit Wasser nicht mischbar ist (z.B. Benzol, Chloroform oder Dichlormethan), trennt ab, trocknet die organische Phase über Natriumsulfat, filtriert, dampft ein und reinigt durch Chromatographie und/oder Kristallisation. Das Produkt kann auch durch Kristallisation eines seiner Säureadditionssalze gereinigt werden.

Beispiel 1

Man kocht ein Gemisch von 27,1 g 1-p-Chlorphenoxy-2-chlor-3-(1-imidazolyl)-propan (erhältlich durch Umsetzung von Epichlorhydrin mit Na-p-chlorphenolat zu 1-p-Chlorphenoxy-2,3-epoxypropan, Reaktion mit Imidazol zu 1-p-Chlorphenoxy-3-(1-imidazolyl)-propan-2-ol und Umsetzung mit $SOCl_2$), 17,9 g 2,6-Dichlorthiophenol und 7 g $K_2CO_3$ in 1 l Aceton 4 Stunden unter Rühren , dampft ein, arbeitet wie üblich auf und erhält 1-p-Chlorphenoxy-2-(2,6-dichlorphenylthio)-3-(1-imidazolyl)-propan. Hydrochlorid (Ia), F. 175-177°.

Analog sind erhältich:

1-Phenoxy-2-(2,6-dichlorphenylthio)-3-(1-imidazolyl)-propan, Hydrochlorid, F. 168 - 170°
1-o-Fluorphenoxy-2-(2,6-dichlorphenylthio)-3-(1-imidazolyl)-propan
1-m-Fluorphenoxy-2-(2,6-dichlorphenylthio)-3-(1-imidazolyl)-propan
1-p-Fluorphenoxy-2-(2,6-dichlorphenylthio)-3-(1-imidazolyl)-propan, Hydrochlorid, F. 153 - 155°
1-o-Chlorphenoxy-2-(2,6-dichlorphenylthio)-3-(1-imidazolyl)-propan, Hydrochlorid, F. 179 - 181°
1-m-Chlorphenoxy-2-(2,6-dichlorphenylthio)-3-(1-imidazolyl)-propan
1-o-Bromphenoxy-2-(2,6-dichlorphenylthio)-3-(1-imidazolyl)-propan
1-m-Bromphenoxy-2-(2,6-dichlorphenylthio)-3-(1-imidazolyl)-propan
1-p-Bromphenoxy-2-(2,6-dichlorphenylthio)-3-(1-imidazolyl)-propan

1-p-Jodphenoxy-2-(2,6-dichlorphenylthio)-3-(1-imidazolyl)-propan

1-(2,4-Difluorphenoxy)-2-(2,6-dichlorphenylthio)-3-(1-imidazolyl)-propan

1-(2,3-Dichlorphenoxy)-2-(2,6-dichlorphenylthio)-3-(1-imidazolyl)-propan

1-(2,4-Dichlorphenoxy)-2-(2,6-dichlorphenylthio)-3-(1-imidazolyl)-propan, Nitrat, F. 134 - 136°

1-(2,5-Dichlorphenoxy)-2-(2,6-dichlorphenylthio)-3-(1-imidazolyl)-propan

1-(2,6-Dichlorphenoxy)-2-(2,6-dichlorphenylthio)-3-(1-imidazolyl)-propan

1-(3,4-Dichlorphenoxy)-2-(2,6-dichlorphenylthio)-3-(1-imidazolyl)-propan

1-(3,5-Dichlorphenoxy)-2-(2,6-dichlorphenylthio)-3-(1-imidazolyl)-propan

1-(2,4-Dibromphenoxy)-2-(2,6-dichlorphenylthio)-3-(1-imidazolyl)-propan

1-Phenylthio-2-(2,6-dichlorphenylthio)-3-(1-imidazolyl)-propan

1-o-Chlorphenylthio-2-(2,6-dichlorphenylthio)-3-(1-imidazolyl)-propan

1-p-Chlorphenylthio-2-(2,6-dichlorphenylthio)-3-(1-imidazolyl)-propan, Hydrochlorid, F. 150 - 152°

1,2-Bis-(2,6-dichlorphenylthio)-3-(1-imidazolyl)-propan

1-Phenoxy-2-(2,6-dichlorphenylthio)-3-(2-methyl-1-imidazolyl)-propan

1-o-Fluorphenoxy-2-(2,6-dichlorphenylthio)-3-(2-methyl-1-imidazolyl)-propan

1-m-Fluorphenoxy-2-(2,6-dichlorphenylthio)-3-(2-methyl-1-imidazolyl)-propan

1-p-Fluorphenoxy-2-(2,6-dichlorphenylthio)-3-(2-methyl-1-imidazolyl)-propan

1-o-Chlorphenoxy-2-(2,6-dichlorphenylthio)-3-(2-methyl-1-imidazolyl)-propan

1-m-Chlorphenoxy-2-(2,6-dichlorphenylthio)-3-(2-methyl-1-imidazolyl)-propan

1-p-Chlorphenoxy-2-(2,6-dichlorphenylthio)-3-(2-methyl-1-imidazolyl)-propan, Hydrochlorid, F. 162 - 163°

1-o-Bromphenoxy-2-(2,6-dichlorphenylthio)-3-(2-methyl-1-imidazolyl)-propan

1-m-Bromphenoxy-2-(2,6-dichlorphenylthio)-3-(2-methyl-1-imidazolyl)-propan

1-p-Bromphenoxy-2-(2,6-dichlorphenylthio)-3-(2-methyl-1-imidazolyl)-propan

1-p-Jodphenoxy-2-(2,6-dichlorphenylthio)-3-(2-methyl-1-imidazolyl)-propan

1-(2,4-Difluorphenoxy)-2-(2,6-dichlorphenylthio)-3-2-methyl-imidazolyl)-propan

1-(2,3-Dichlorphenoxy)-2-(2,6-dichlorphenylthio)-3-(1-imidazolyl)-propan

1-(2,4-Dichlorphenoxy)-2-(2,6-dichlorphenylthio)-3-(2-methyl-1-imidazolyl)-propan

1-(2,5-Dichlorphenoxy) -2-(2,6-dichlorphenylthio)-3-(2-methyl-1-imidazolyl)-propan

1-(2,6-Dichlorphenoxy)-2-(2,6-dichlorphenylthio)-3-(2-methyl-1-imidazolyl)-propan

1-(3,4-Dichlorphenoxy)-2-(2,6-dichlorphenylthio)-3-(2-methyl-1-imidazolyl)-propan

1-(3,5-Dichlorphenoxy)-2-(2,6-dichlorphenylthio)-3-(2-methyl-1-imidazolyl)-propan

1-(2,4-Dibromphenoxy)-2-(2,6-dichlorphenylthio)-3-(2-methyl-1-imidazolyl)-propan

1-Phenylthio-2-(2,6-dichlorphenylthio)-3-(1-imidazolyl)-propan

1-o-Chlorphenylthio-2-(2,6-dichlorphenylthio)-3-(2-methyl-1-imidazolyl)propan

1-p-Chlorphenylthio-2-(2,6-dichlorphenylthio)-3-(2-methyl-1-imidazolyl)-propan

1,2-Bis-(2,6-dichlorphenylthio)-3-(2-methyl-1-imidazolyl)-propan.

Beispiel 2

Ein Gemisch von 44,2 g 1-p-Chlorphenoxy-2-methylsulfonyloxy-3-(2,6-dichlorphenylthio)-propan /erhältlich durch Reaktion von 1-p-Chlorphenoxy-2,3-epoxypropan mit Na-2,6-dichlorthiophenolat zu 1-p-Chlorphenoxy-3-(2,6-dichlorphenylthio)-propan-2-ol und Umsetzung mit Methansulfonylchlorid/, 20,4 g Imidazol und 250 ml Dimethylformamid wird 2 Stunden auf 100° erhitzt und dann wie üblich aufgearbeitet. Man erhält Ia, F. 175 - 177°.

Analog sind die übrigen in Beispiel 1 genannten Imidazolderivate erhältlich.

Beispiel 3

Man erhitzt 38,2 g 1-Chlor-2-(2,6-dichlorphenylthio)-3-p-chlorphenoxy-propan /erhältlich durch Reaktion von 3-Brompropionsäure mit Na-p-chlorphenolat zu 3-p-Chlorphenoxypropionsäure, Bromierung zu 2-Brom-3-p-chlorphenoxypropionsäure, Reaktion mit Na-2,6-dichlor-thiophenolat zu 2-(2,6-Dichlorphenylthio)-3-p-chlorphenoxy-propionsäure, $LiAlH_4$-Reduktion zum Alkohol und Umsetzung mit $SOCl_2$/ mit 40 g Imidazol 5 Stunden auf 120°, kühlt ab, arbeitet wie üblich auf und erhält Ia, F. 175-177°.

Analog sind die übrigen in Beispiel 1 genannten Imidazolderivate erhältlich.

Beispiel 4

Ein Gemisch aus 12,9 g p-Chlorphenol, 36,6 g 1-Brom-2-(2,6-dichlorphenylthio)-3-(1-imidazolyl)-propan /erhältlich durch Reaktion von 3-Brompropionsäure mit Imidazol zu 3-(1-Imidazolyl)-propionsäure, Bromierung zu 2-Brom-3-(1-imidazolyl)-propionsäure, Reaktion mit Na-2,6-dichlorthiophenolat zu 2-(2,6-Dichlorphenylthio)-3-(1-imidazolyl)-propionsäure, Reduktion mit $LiAlH_4$ zum entsprechenden Alkohol und Umsetzung mit $PBr_3$/, 8 g NaOH und 400 ml Ethanol wird 10 Stunden auf 100° erhitzt. Man dampft ein, arbeitet wie üblich auf und erhält Ia, F. 175 - 177°.

Analog sind die übrigen in Beispiel 1 genannten Imidazolderivate erhältlich.

Die nachstehenden Beispiele betreffen pharmazeutische Zubereitungen, die Imidazolderivate der Formel I oder ihre Säureadditionssalze enthalten:

Beispiel A: Tabletten

Ein Gemisch von 1 kg Ia, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 100 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

10 kg Ia werden in üblicher Weise in Hartgelatinekapseln gefüllt, so daß jede Kapsel 50 mg Wirkstoff enthält.

Beispiel D: Ampullen

Eine Lösung von 1 kg Ia in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Beispiel E: Salbe

Man löst 2 kg Ia in einem warmen verflüssigtem Gemisch von 40 kg Polyethylenglykol 400 und 58 kg Polyethylenglykol 1500. Die Lösung wird während des Kühlens gerührt und als Salbe zur Behandlung von Pilz- und Bakterieninfektionen verwendet.

Beispiel F: Creme

Man erwärmt in üblicher Weise ein Gemisch aus 20 kg Ia, 200 kg Polyethylenglykol 1000-monocetylether, 50 kg Polyethylenglykol 1500-mono-cetylether, 150 kg Vaseline, 50 kg Paraffinöl und 2 kg Sorbinsäure, läßt abkühlen und rührt 528 kg Wasser ein.

Beispiel G: Creme

Man erwärmt ein Gemisch aus 2 kg Ia, 5 kg 1,2-Propandiol, 5 kg Glycerinstearat, 5 kg Walrat, 10 kg Isopropylmyristat und 4 kg Polysorbat 60, läßt abkühlen und rührt 69 kg Wasser ein.

Beispiel H: Lösung

2 kg Ia werden in 98 kg 1,2-Propandiol gelöst. Die Lösung wird zur Behandlung von Pilz- und Bakterieninfektionen verwendet.

Beispiel I: Spray:

Der Spray besteht aus einer Lösung von 1 (Gewichtsteil) Ia, 10 Isopropylmyristat, 15 Paraffinöl, 30 Ethanol und 44 Isopropanol.

Analog sind Tabletten, Dragees, Kapseln, Ampullen, Salben, Cremes, Lösungen und Sprays erhältlich, die einen oder mehrere der übrigen Wirkstoffe der Formel I und/oder ihrer physiologisch unbedenklichen Salze enthalten.

Merck Patent Gesellschaft
mit beschränkter Haftung
D a r m s t a d t

Patentansprüche:

1. Schwefelhaltige Imidazolderivate der allgemeinen Formel I

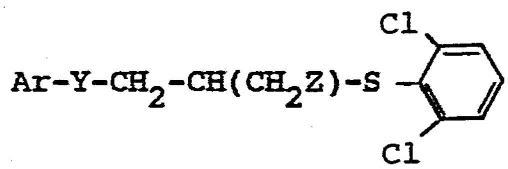 I

worin

Ar einen unsubstituierten oder einen durch ein oder zwei Halogenatome substituierten Phenylrest,

Y O oder S und

Z einen 1-Imidazolyl- oder einen 2-Methyl-1-imidazolylrest

bedeuten,

sowie ihre Säureadditionssalze.

2. 1-p-Chlorphenoxy-2-(2,6-dichlorphenylthio)-3-(1-imidazolyl)-propan.

3. Verfahren zur Herstellung von schwefelhaltigen Imidazolderivaten der allgemeinen Formel I nach Anspruch 1 sowie von ihren Säureadditionssalzen, dadurch gekennzeichnet, daß man eine Verbindung der allgemeinen Formel II

$Ar-Y-CH_2-CH(CH_2Z)-X$ II

worin

X Cl, Br, J, OH oder funktionell abgewandeltes OH bedeutet und

Ar, Y und Z die angegebenen Bedeutungen haben

mit 2,6-Dichlorthiophenol oder einem seiner Salze umsetzt,

oder daß man eine Verbindung der allgemeinen Formel III

$Ar-Y-CH_2-CH(CH_2X^1)-X^2$ III

worin

der eine der Reste $X^1$ und $X^2$ X,

der andere dieser Reste - S 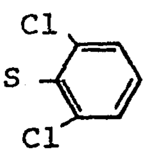 bedeutet und

Ar, X und Y die angegebenen Bedeutungen haben,

mit einem Imidazol der allgemeinen Formel H-Z (IV) oder einem seiner Metallderivate umsetzt,

oder daß man eine Verbindung der allgemeinen Formel V

$X-CH_2-CH(CH_2Z)-S$ 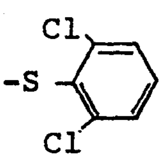 V

worin

X und Z die angegebenen Bedeutungen haben,

mit einer Verbindung der allgemeinen Formel VI

Ar-Y-H VI

worin
Ar und Y die angegebenen Bedeutungen haben

oder einem ihrer Salze umsetzt

und/oder daß man, falls erwünscht, eine Base der allgemeinen Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.